(19) Europäisches Patentamt
European Patent Office
Office européen des brevets

(11) **EP 1 245 185 B1**

(12) **EUROPÄISCHE PATENTSCHRIFT**

(45) Veröffentlichungstag und Bekanntmachung des Hinweises auf die Patenterteilung:
**11.07.2007   Patentblatt 2007/28**

(51) Int Cl.:
*A61B 5/0452* (2006.01)   *G06F 17/00* (2006.01)
*A61N 1/365* (2006.01)

(21) Anmeldenummer: **02090115.3**

(22) Anmeldetag: **19.03.2002**

(54) **Verfahren und Vorrichtung zur Charakterisierung des Myokardzustands**

Method and apparatus for characterising the condition of the myocardium

Procédé et appareil de caractérisation de la condition du myocarde

(84) Benannte Vertragsstaaten:
**AT BE CH CY DE DK ES FI FR GB GR IE IT LI LU MC NL PT SE TR**

(30) Priorität: **21.03.2001   DE 10114724**

(43) Veröffentlichungstag der Anmeldung:
**02.10.2002   Patentblatt 2002/40**

(73) Patentinhaber: **BIOTRONIK GmbH & Co. KG**
**12359 Berlin (DE)**

(72) Erfinder:
• **Anosov, Oleg**
  **91054 Erlangen (DE)**
• **Berdychev, Serguej**
  **91054 Erlangen (DE)**
• **Hensel, Bernhard**
  **91056 Erlangen (DE)**
• **Khassanov, Ildar**
  **91058 Erlangen (DE)**
• **Schaldach, Max**
  **verstorben (DE)**

(74) Vertreter: **Lindner-Vogt, Karin L.**
**BIOTRONIK GmbH & Co. KG**
**Corporate Intellectual Properties**
**Woermannkehre 1**
**12359 Berlin (DE)**

(56) Entgegenhaltungen:
EP-A- 0 947 215            WO-A-00/45700
US-A- 5 351 696           US-A- 5 497 780
US-A- 5 623 936

# EP 1 245 185 B1

**Beschreibung**

Die vorliegende Erfindung betrifft eine Vorrichtung zur Charakterisierung eines Zustandes eines Myokards mit einem Erregungswellendetektor, der dazu ausgebildet ist, eine sich durch das Myokard ausbreitende, elektrische Erregungswelle an einem ersten und zweiten Punkt des Myokards als erstes und zweites Signal zu erfassen, und mit einer Analyseeinrichtung, die mit dem Erregungswellendetektor verbunden und ausgebildet ist, das erste und zweite Signal zu analysieren. Ferner betrifft die Erfindung ein Verfahren zum Betreiben einer Analysevorrichtung mit dem Schritt Erfassen einer elektrischen Erregungswelle an einem ersten und zweiten Punkt des Myokards als erstes und zweites Signal.

Die Charakterisierung des Myokardzustands ist ein wichtiges Anliegen, denn das Myokard bzw. der Herzmuskel sorgt für eine periodische Kontraktion der Herzkammer und damit gewährleistet er die notwendige Durchblutung des Körpers. Störungen oder Defekte im Myokard führen zu einer Beeinträchtigung der Pumpleistung des Herzens und letztlich kann ein solcher Defekt zu einem Herzstillstand führen.

Das regelmäßige Schlagen des Herzens ist auf die inhärente Rhythmizität der Herzmuskulatur zurückzuführen. Im Herzen befinden sich keine steuernden Nerven und ein äußerer Regelmechanismus ist nicht notwendig, um den Herzmuskel rhythmisch kontrahieren zu lassen. Der Rhythmus des Herzschlages entstammt dem Herzen selber. Es kann beispielsweise unter Laborbedingungen gezeigt werden, dass Fragmente der Herzmuskulatur fortfahren rhythmisch zu kontrahieren. Diese intensische Fähigkeit ist jedoch nicht ausreichend, um ein effizientes Funktionieren des Herzens zu ermöglichen. Dazu ist es notwendig, die Muskelkontraktion im Herzen zu koordinieren. Dies geschieht mittels eines Leitungssystems im Herzen, das hauptsächlich aus zwei, aus spezialisiertem Gewebe bestehenden Knoten besteht, von denen Impulse ausgehen, und einem Leitungssystem zur Übertragung dieser Impulse, dessen Enden bis zur Innenfläche der Ventrikel reicht. Die Rate, mit der das Herz kontrahiert, und die Synchronisation von atrialer und ventriklärer Kontraktion, die für ein effektives Pumpen von Blut notwendig ist, hängt von den elektrischen Eigenschaften der Myokardzellen und der Leitung von elektrischen Impulsen von einer Region des Herzens in eine andere ab. Die Charakterisierung dieser Erregungsleitung gibt somit Aufschluss über den Zustand des Myokards.

Die Charakterisierung der Leitungseigenschaft des Myokards erfolgt herkömmlicher Weise dadurch, dass an zwei Punkten des Myokards dessen elektrisches Potenzial gemessen wird. Eine Erregungswelle, die sich zwischen diesen beiden Punkten ausbreitet, erzeugt die an den beiden Punkten aufgenommenen Signale. Diese Signale geben demnach das Eintreffen der Erregungswelle an dem ersten und zweiten Punkt wieder. Schließlich werden die beiden aufgenommenen Signale mittels einer Analyseeinrichtung miteinander verglichen. So kann aus dem zeitlichen Abstand zwischen dem Auftreten der Signale und der Entfernung der Punkte im Myokard, wo die Signale aufgenommen wurden, die Ausbreitungsgeschwindigkeit der Erregungswelle zwischen den beiden Messpunkten im Myokard ermittelt werden. Eine Störung oder Veränderung der Leitungseigenschaften des Myokards kommt beispielsweise dadurch zum Ausdruck, dass sich der zeitliche Abstand zwischen dem aufgenommenen Signal verändert, oder ein zweites Signal nach Aufnahme eines ersten Signals nicht gemessen werden kann. Letzteres spricht dafür, dass die Erregungsleitung zwischen den beiden Punkten gänzlich unterbrochen ist.

Aus EP 0 947 215 ist eine Vorrichtung bekannt, die mittels jeweils einer im linken und im rechten Atrium angeordneten Elektrode eine interatriale Überleitungszeit als körpereigenes Laufzeitsignal ermittelt.

Die vorstehend erwähnten, herkömmlichen Vorrichtungen zur Charakterisierung eines Myokardzustands lassen jedoch nur wenige Rückschlüsse auf den Zustand des Myokards zu, denn sie berücksichtigen lediglich die Ausbreitungsgeschwindigkeit und die Abschwächung der sich ausbreitenden Erregungswelle. Es ist jedoch anzunehmen, dass die aufgenommenen Signale eine Vielzahl von Informationen liefern können, die Aufschluss über den Zustand des Myokard geben können.

Es ist daher Aufgabe der vorliegenden Erfindung, eine Vorrichtung und ein Verfahren zur Charakterisierung eines Zustands eines Myokards bereitzustellen, die es erlauben den Einfluss des Myokards auf die Ausbreitung von Erregungswellen genauer zu charakterisieren.

Die Aufgabe wird gelöst durch eine Vorrichtung zur Charakterisierung eines Zustands eines Myokards mit einem Erregungswellen-Detektor, der dazu ausgebildet ist, eine sich durch das Myokard ausbreitende, elektrische Erregungswelle an einem ersten ($r_1$) und zweiten Punkt ($r_2$) des Myokards als erstes Signal ($S_1(t)$) und zweites Signal ($S_2(t)$) zu erfassen, und einer Analyseeinrichtung, die mit dem Erregungswellen-Detektor verbunden und ausgebildet ist, das erste ($S_1(t)$) und zweite Signal ($S_2(t)$) zu analysieren, wobei die Analyseeinrichtung ausgebildet ist, einen Unterschied zwischen den Signalformen des ersten ($S_1(t)$) und zweiten Signals ($S_2(t)$) zu erfassen.

Die Erfindung stellt somit auf die Erfassung und den Vergleich der Signalmorphologieen einer, an zwei unterschiedlichen Orten erfassten und daher zwei Signale liefernden Erregungswelle ab. Vorteilhaft an der erfindungsgemäßen Vorrichtung ist insbesondere, dass sie es erlaubt, den Einfluss des Myokardzustands auf die Form einer sich ausbreitenden Erregungswelle zu charakterisieren. Es ist anzunehmen, dass spezifische Eigenschaften des Myokards einen Einfluss auf die Formveränderung haben, so dass der erfasste Unterschied zwischen den Signalformen eine Charakterisierung des Myokardzustands erlaubt. Bei der Ausbreitung der Erregungswelle "verläuft" diese, das heißt es kommt zu einer Dispersion des sich ausbreitenden Signals, die durch Vergleich der an zwei verschiedenen Orten aufgenommenen Signalform bzw. Signalmorphologieen ermittelt werden und weiter ausgewertet werden kann.

Die Analyseeinrichtung umfaßt vorzugsweise mindestens eine Parametereinheit, die ausgebildet ist, die Signalform des ersten ($S_1(t)$) und zweiten Signals ($S_2(t)$) anhand zumindest eines Parameters zu charakterisieren. Der Unterschied zwischen den Werten des jeweils für das erste und zweite Signal ermittelten Parameter ist dann ein geeignetes Maß für die charakteristische Veränderung der Form der erfaßten Erregungswelle zwischen den Punkten $r_1$ und $r_2$. Ein solcher Parameter kann beispielsweise die Halbwertsbreite oder die maximale Steigung der an den Punkten $r_1$ und $r_2$ erfaßten Signale darstellen. Es ist selbstverständlich auch möglich die Signalform mittels mehrerer unterschiedlicher Parameter zu charakterisieren und diese miteinander zu vergleichen.

Zur Analyse der jeweiligen Signalform werden vorzugsweise sowohl das erste als auch das zweite Signal durch eine Überlagerung einer Menge von Funktionen dargestellt. Daher ist die Analyseeinrichtung bevorzugt dazu ausgebildet, das erste ($S_1(t)$) und zweite Signal ($S_2(t)$) durch eine Überlagerung einer Menge von Funktionen {f(wt)} mit $w \in R$ darzustellen, wobei $S_1(t) = \int_{-\infty}^{\infty} C_1(w)f(wt)dw$ und $S_2(t) = \int_{-\infty}^{\infty} C_2(w)f(wt)dw$ entspricht.

Die gewählte Funktionenschar {f(wt)} muss geeignet sein, die aufgenommenen Signale darzustellen. Dass solche Darstellungen existieren, kann mathematisch nachgewiesen werden und ist bekannt. Die Darstellbarkeit der aufgenommenen Signale beruht auf der Tatsache, dass sie quadratintegrabel sind, das heißt dass die Fläche unter den quadrierten Signalen endlich ist. Die Menge der quadratintegrablen Funktionen bildet einen Vektorraum. Wenn die Funktionenschar {f(wt)} eine Basis dieses Vektorraumes bildet, so kann jedes beliebige Signal durch diese Funktionen dargestellt werden. Jede Funktionenschar die den Vektorraum der quadratintegrablen Funktion aufspannt, ist somit dazu geeignet, die aufgenommen Signale darzustellen. Der Vorteil einer solchen Darstellung liegt darin, dass sich eine Veränderung der Signalform in einer Veränderung der Funktionen Cj(w)f(wt) ($_i$=1,2) ausdrückt. Der Einfluss des Myokards auf die Ausbreitung der Erregungswelle kann somit als Beeinflussung oder Veränderung jeder einzelnen Funktion der Funktionenschar interpretiert werden.

In einer ersten Ausführungsvariante besitzt die Analyseeinrichtung eine Fourier-Analyse-Einheit. Diese ist dazu ausgebildet, eine Fourier-Analyse durchzuführen, das heißt für die Funktionen f(wt) werden die Exponentialfunktionen exp(iwt) eingesetzt. Die Koeffizienten $C_j(w)$ (j=1,2) errechnen sich dann gemäß $C_j(w) = \int S_j(t)\exp(-iwt)dt$. Die vorstehenden Integrale werden auch als FourierTransformation bezeichnet. Die Koeffizienten sind also im Allgemeinen komplexwertig. Die Signale $S_j(t)$ (j=1,2) lassen sich somit mittels $S_j(t) = \int_{-\infty}^{\infty} C_j(w)\exp(iwt)dw$ darstellen, was äquivalent zu $S_j(t) = \int_{-\infty}^{\infty} D_j(w)\cos(wt + e_j(w))dw$ ist. $D_j(w)$ stellt das Amplitudenspektrum und $e_j(w)$ stellt das Phasenspektrum des aufgenommenen Signals dar. Somit können Unterschiede in der Signalform des ersten (j=1) und zweiten (j=2) Signals als Dämpfung und Phasenverschiebung einer Fourier-Komponente $C_j(w)\exp(-iwt)$ interpretiert werden. Die Analyseeinrichtung umfasst eine Dämpfungsanalyseeinheit, die mit der Fourier-Analyse-Einheit verbunden ist und ausgebildet ist eine Dämpfung δ(w) einer Fourier-Kompenenten zwischen den Punkten $r_1$ und $r_2$ zu ermitteln. Ferner besitzt die Analyseeinrichtung bevorzugt eine Geschwindigkeitsanalyseeinheit, die mit der Fourier-Analyse-Einheit verbunden und ausgebildet ist, eine Phasengeschwindigkeit $v_p(w)$ einer Fourier-Komponenten zu ermitteln. Die Phasengeschwindigkeit bezeichnet die Geschwindigkeit mit der sich eine Fourier-Komponente durch das Myokard ausbreitet. Die Geschwindigkeit kann für alle Fourier-Komponenten gleich oder für jede der Fourier-Komponenten verschieden sein. Letzteres führt zu einer Änderung der Signalform und wird als Dispersion bezeichnet. Die Phasengeschwindigkeit lässt sich anhand der Phasenänderung e(w) zwischen der entsprechenden Fourier-Komponente des ersten und zweiten Signals ermitteln. Die Phasenverschiebung ist umso größer je weiter die Messpunkte auseinander liegen und umso kleiner je größer die Phasengeschwindigkeit ist.

Die vorstehend beschriebene Fourier-Analyse ist nur eine von vielen möglichen Darstellungsweisen für die aufgenommenen Signale. Ferner ist es möglich, die aufgenommenen Signale mittels einer Wavelet-Analyse darzustellen. In einer zweiten Ausführungsvariante umfasst die Analyseeinrichtung eine Wavelet-Analyseeinheit. Die Wavelet-Transformierte eines Signals S(t) ist durch $C(a,b) = \int_{-\infty}^{\infty} a^{-1/2}S(t)\Psi((t-b)/a)dt$ gegeben. Im Unterschied zur Fouriertransformierten ist die Wavelet-Transformierte C(a,b) eine Funktion von zwei verschiedenen Parametern a und b. Die Funktion $\Psi((t-b)/a)$ stellt ein sogenanntes Wavelet dar. Dieses ist definitionsgemäß so gewählt, dass man mittels der Transformation

$$S(t) = \sum_{k=-\infty}^{\infty} \int_{-\infty}^{\infty} a_k^{-1/2} C(a_k, b) \Psi\left(\frac{t-b}{a_k}\right) db \text{, (wobei } a_k = 2^k)$$

wieder das ursprüngliche Signal S(t) erhält. Die Wavelet-Komponenenten $s_a(t)$ lassen sich wie folgt definieren

$$s_a(t) = \int_{-\infty}^{\infty} a^{-1/2} C(a, b) \Psi\left(\frac{t-b}{a}\right) db .$$

Das ursprüngliche Signal S(t) lässt sich als Überlagerung dieser Wavelet-Komponenten $s_a(t)$ darstellen. Diese Darstellung lässt sich für die am Punkt $r_1$ und $r_2$ aufgenommenen Signale jeweils ermitteln, wobei sich die Wavelet-Komponenten für die unterschiedlichen Signale an den Punkten $r_1$ und $r_2$ voneinander unterscheiden..

In der zweiten Ausführungsvariante umfasst die erfindungsgemäße Vorrichtung eine Dämpfungsanalyseeinheit, die mit der Wavelet-Analyseeinheit verbunden ist und ausgebildet ist eine Dämp-

$$\text{fung} \delta(a) = \int_{-\infty}^{\infty} s_{a,2}^2(t) dt \Big/ \int_{-\infty}^{\infty} s_{a,1}^2(t) dt \quad \text{der Wavelet-Komponente } s_{a,1}(t) \text{ und } s_{a,2}(t) \text{ zwischen den Punkten } (r_1 \text{ und}$$

$r_2$) zu ermitteln (Mit $s_{a,1}(t)$ und $s_{a,2}(t)$ ist die Funktion $s_a(t)$ für die Punkte $r_1$ und $r_2$ bezeichnet). Die Wavelet-Komponenten der an den Punkten $r_1$ und $r_2$ aufgenommenen Signale sind jeweils Funktionen der Zeit t und eines Parameters a. Diejenigen Wavelet-Komponenten, die denselben Parameterwert a aufweisen, werden miteinander identifiziert und für diese eine Dämpfung zwischen den Punkten $r_1$ und $r_2$ ermittelt.

Ferner ist die Geschwindigkeitsanalyseeinheit vorzugsweise mit der Wavelet-Analyseeinheit verbunden und ausgebildet, eine Phasengeschwindigkeit $v_p(a)$ der Wavelet-Komponente $s_a(t)$ zu ermitteln. Dies erfolgt dadurch, dass der Quotient aus dem Abstand zwischen den Punkten $r_1$ und $r_2$ und den Nulldurchgängen $t_{a,2}$ und $t_{a,1}$ der Wavelet-Komponenten ermittelt wird.

Ferner kann die Geschwindigkeitsanalyseeinheit dazu ausgebildet sein, eine Gruppengeschwindigkeit $v_g(a)$ der Wavelet-Komponente $s_a(t)$ zu ermitteln. Dazu werden zunächst jeweils die Einhüllenden $A_{a,1}(t)$ und $A_{a,2}(t)$ der Wavelet-Komponente $s_a(t)$ der Signale $S_1(t)$ und $S_2(t)$ berechnet. Die Einhüllenden $A_{a,1}(t)$ und $A_{a,2}(t)$ lassen sich anhand von $A_{a,j} = [s_{a,j}^2(t) + \hat{s}_{a,j}^2(t)]^{1/2}$ (j=1,2) berechnen, wobei $\hat{s}_{a,j}^2(t)$ durch die Hilbert-Transformation $\hat{s}_{a,j}(t) = -\pi^{-1} \int_{-\infty}^{\infty} \frac{s_{a,j}(t)}{\tau - t} d\tau$ gegeben ist. Die Einhüllenden der Wavelet-Komponente $s_a(t)$ der Signale $S_1(t)$ und

$S_2(t)$ besitzen jeweils ein Maximum zu den Zeitpunkten $\tau_{a,2}$ und $\tau_{a,1}$. Die Gruppengeschwindigkeit der Wavelet-Komponente ergibt sich dann aus dem Abstand zwischen dem Ort $r_1$ und $r_2$ der aufgenommenen Signale und dem zeitlichen Abstand zwischen den Maxima der Einhüllenden der Wavelet-Komponente der Signale $S_1(t)$ und $S_2(t)$.

Schließlich besitzt die Analyseeinrichtung der erfindungsgemäßen Vorrichtung vorzugsweise eine Refraktionsindex-Analyse-Einheit, die einen zu dem Refraktionsindex $n(a) = v_g(a)/v_p(a)$ der Optik analogen Wert ermittelt. Die Refraktionsindex-Analyse-Einheit ist mit der Geschwindigkeitsanalyse-Einheit verbunden und ausgebildet, einen Quotienten aus Gruppengeschwindigkeit und Phasengeschwindigkeit einer Wavelet-Komponente zu ermitteln.

Aus dem Refraktionsindex n(a) und der Dämpfung $\delta(a)$ einer Wavelet-Komponente lässt sich in Analogie zur Optik ein komplexer Refraktionsindex einer Wavelet-komponente als durch $n(a)+i\delta(a)$ ermitteln.

Ferner umfasst der Erregungswellendetektor der erfindungsgemäßen Vorrichtung eine erste und zweite Elektrode zum Erfassen des ersten Signals $S_1(t)$ und $S_2(t)$, die zu dem geeignet sind, endokardial oder epikardial platziert zu werden.

Vorzugsweise umfasst die Vorrichtung einen Signalspeicher, der mit dem Erregungswellendetektor und der Analyseeinrichtung verbunden ist und ausgebildet ist das erste und zweite Signal zwischenzuspeichern. Die Erfassung und Analyse der aufgenommenen Signale kann somit zeitlich voneinander getrennt durchgeführt werden.

In einem Beispiel, das nicht Bestandteil der beanspruchten Erfindung ist die Analyseeinrichtung ausgebildet, das erste und zweite Signal separat für jeden Herzzyklus durch die Überlagerung der Menge der Funktion {f(wt)} darzustellen. Die aufgenommenen Signale geben somit lediglich eine sich ausbreitende Erregungswelle an zwei Punkten $r_1$ und $r_2$ wieder. Ein bevorzugtes Ausführungsbeipiel der vorliegenden Erfindung wird anhand der beigefügten Figuren erläutert. Es zeigen:

Figur 1:     ein Blockschaltbild eines Ausführungsbeispiels der erfindungsgemäßen Vorrichtung zur Charakterisierung eines Zustands eines Myokards;

Figur 2:     eine schematische Ansicht der Anordnung der Elektroden des ersten Ausführungsbeispiels zur Aufnahme von elektrischen Signalen aus dem Myokard;

Figur 3:     eine Wavelet-Komponente $s_a(t)$ und deren Einhüllende $A_a(t)$;

Figur 4:     zwei zeitgleich an unterschiedlichen Punkten des Myokards aufgenommene Wavelet-Komponenten sowie die zeitliche Verschiebung deren Nulldurchgänge, und

Figur 5:     die Einhüllenden der zwei zeitgleich an unterschiedlichen Punkten des Myokards aufgenommenen Wavelet-Komponenten sowie die zeitliche Verschiebung deren Maxima.

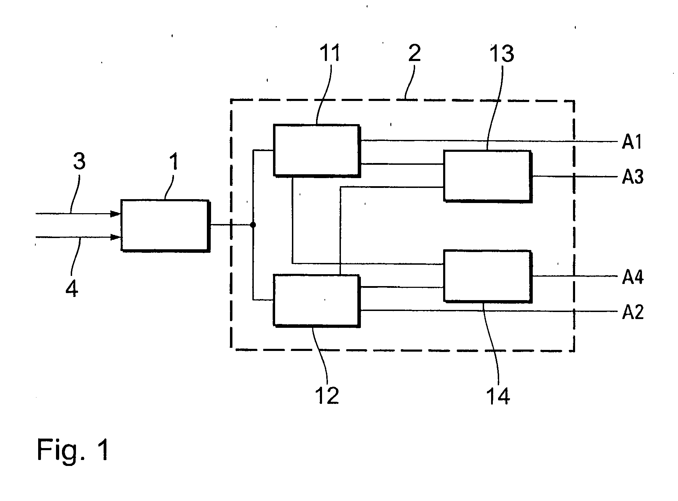

Im Folgenden wird das in Figur 1 gezeigte Blockschaltbild der erfindungsgemäßen Vorrichtung zur Charakterisierung eines Myokardzustandes beschrieben. Die Vorrichtung umfasst einen Erregungswellendetektor 1 und eine Analyseeinrichtung 2, die miteinander verbunden sind. Der Erregungswellendetektor 1 umfasst zwei Leitungen 3 und 4 zum Aufnehmen von Signalen aus dem Myokard. Die zeitgleich aufgenommenen Signale werden von dem Erregungswellendetektor 1 erfasst und an die Analyseeinrichtung 2 weitergeleitet. Diese besitzt entweder eine Fourier-Analyse-Einheit 11 oder eine Wavelet-Analyseeinheit 12 oder sowohl eine Fourier-Analyse-Einheit 11 als auch eine Wavelet-Analyse-Einheit 12. Die aufgenommenen Signale können somit entweder einer Fourier-Analyse oder einer Wavelet-Analyse oder sowohl einer Fourier- als auch einer Wavelet-Analyse unterzogen werden. Die Fourier-Analyseeinheit 11 bewirkt, dass das aufgenommene Signal nach seinen spektralen Bestandteilen entwickelt wird. Das Ergebnis dieser Analyse, d. h. das Frequenzspektrum des aufgenommenen Signals, kann über einen Ausgang A1 ausgegeben werden. Die Wavelet-Analyseeinheit 12 entwickelt die aufgenommenen Signale hingegen nach ihren Wavelet-Komponenten, die über den Ausgang A2 ausgegeben werden können. Die Fourier-Analyseeinheit und die Wavelet-Analyseeinheit sind jeweils mit einer Geschwindigkeitsanalyseeinheit und einer Dämpfungsanalyseeinheit verbunden. Diese sind dazu ausgebildet, die Ausbreitungsgeschwindigkeit bzw. Phasengeschwindigkeit oder Dämpfung der Fourier- bzw. Wavelet-Komponenten zu ermitteln. Die ermittelten Ergebnisse der Geschwindigkeitsanalyseeinheit 13 und Dämpfungsanalyseeinheit 14 werden jeweils über die Ausgänge A3 und A4 ausgegeben.

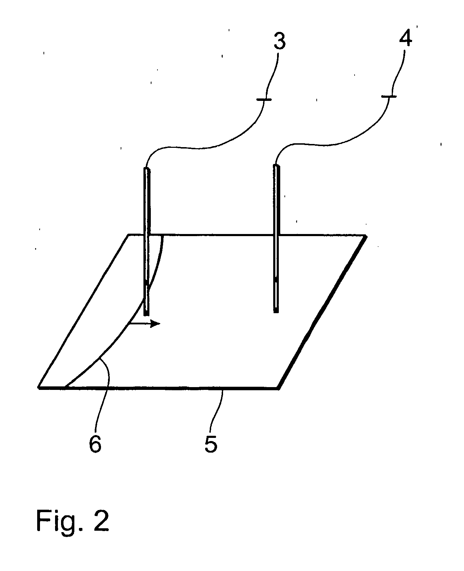

Figur 2 zeigt eine Anordnung der Elektroden zur Aufnahme der Erregungswellensignale an zwei Punkten gemäß dem bevorzugten Ausführungsbeispiel. Dargestellt ist ein Abschnitt des Myokards 5, über den sich eine elektrische Erregungswelle 6 in Pfeilrichtung ausbreitet. Zwei Elektroden 1 und 2 sind voneinander beabstandet an dem Myokard 5 angebracht. Die Erregungswelle 6 breitet sich in Richtung der gedachten Verbindungslinie zwischen Elektrode 1 und 2 aus. Die zeitliche Verzögerung zwischen der Aufnahme der Erregungswelle 6 an der Elektrode 1 und der Elektrode 2 gibt somit Aufschluss über die Ausbreitungsgeschwindigkeit der Erregungswelle 6 in Richtung der Verbindungslinie zwischen Elektroden 1 und 2.

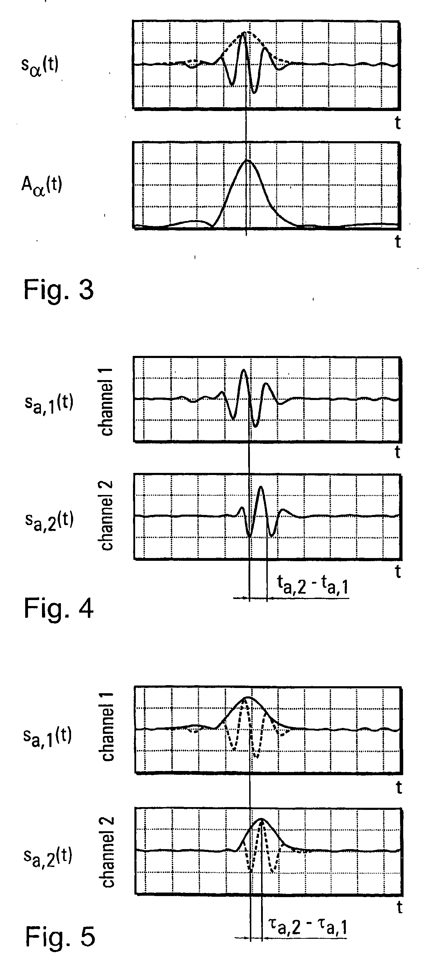

Figur 3 zeigt eine Wavelet-Komponente $s_a(t)$ und deren entsprechende Einhüllende $A_a(t)$. Die Einhüllende ist nochmals in dem oberen Diagramm neben der Wavelet-Komponenten $s_a(t)$ als gestrichelte Linie dargestellt. Sie schmiegt sich an den Kurvenverlauf der Wavelet-Komponenten an und hüllt diesen nach oben hin ein. Die Einhüllende wird für eine Wavelet-Komponente in der Geschwindigkeitsanalyseeinheit 13 ermittelt. Dazu wird zunächst die der Wavelet-

Komponenten $s_a(t)$ Hilbert-konjugierte Funktion $\hat{S}_a(t)$ anhand von $\hat{s}_a(t) = -\pi^{-1} \int_{-\infty}^{\infty} \frac{s_a(t)}{\tau - t} d\tau$ berechnet. Die Einhüllende ergibt sich dann aus

$$A_a(t) = \sqrt{s_a^2(t) + \hat{s}_a^2(t)}$$

Figur 4 zeigt zwei einander entsprechende Wavelet-Komponenten des an der ersten und zweiten Elektrode aufgenommenen Erregungswellensignals. Das mit Channel 1 bezeichnete Diagramm zeigt die Wavelet-Komponente des von der ersten Elektrode aufgezeichneten Signals und das mit Channel 2 bezeichnete Diagramm zeigt wiederum die Wavelet-Komponente des von der zweiten Elektrode aufgenommenen Signals. Zur Ermittlung der Phasengeschwindigkeit der Wavelet-Komponenten wird die zeitliche Verschiebung zwischen den charakteristischen Nulldurchgängen der dargestellten Wavelet-Komponenten ermittelt. Dies geschieht wiederum in der Geschwindigkeitsanalyseeinheit 13. Der zeitliche Unterschied zwischen den Nulldurchgängen ist mit $t_{a,2}$-$t_{a,1}$ bezeichnet. Die Geschwindigkeitsanalyseeinheit 13 ermittelt die Phasengeschwindigkeit der dargestellten Wavelet-Komponenten aus dem Abstand zwischen den Erfassungspunkten $r_1$ und $r_2$, der Elektroden und der vorstehend bezeichneten zeitlichen Verschiebung.

Figur 5 zeigt zwei übereinander angeordnete mit Channel 1 und Channel 2 bezeichnete Diagramme, die jeweils eine Wavelet-Komponente (gestrichelte Linie) mit der entsprechenden Einhüllenden (durchgezogene Linie) darstellen. Channel 1 zeigt die Wavelet-Komponente sowie die zugehörige Einhüllende für das von der ersten Elektrode aufgenommene Signal, während Channel 2 die entsprechenden Kurven für das von der zweiten Elektrode aufgenommene Signal zeigt. Das Maximum der Einhüllenden des ersten Kanals (Channel 1) ist mit $\tau_{a,1}$ bezeichnet und das Maximum der Einhüllenden des zweiten Kanals (Channel 2) ist mit $\tau_{a,2}$ bezeichnet. Die zeitliche Verschiebung zwischen den Maxima der Einhüllenden berechnet sich aus $\tau_{a,2}$-$\tau_{a,1}$. Die Berechnung der zeitlichen Verschiebungen der Maxima der Einhüllenden der entsprechenden Wavelet-Komponenten der ersten und zweiten Elektrode wird von der Geschwindigkeitsanalyseeinheit durchgeführt. Die sogenannte Gruppengeschwindigkeit der Wavelet-Komponenten der Erregungswelle ergibt sich aus dem Quotient zwischen dem Abstand der Messpunkte $r_1$ und $r_2$ und der vorstehend beschriebenen zeitlichen Verschiebung der Maxima der Einhüllenden.

**Patentansprüche**

1. Vorrichtung zur Charakterisierung eines Zustands eines Myokards mit einem Erregungswellen-Detektor (1) der dazu ausgebildet ist, eine sich durch das Myokard ausbreitende, elektrische Erregungswelle an einem erster Punkt $r_1$ und zweiten Punkt $r_2$ des Myokards als erstes Signal $S_1(t)$ und zweites Signal $S_2(t)$ zu erfassen, und einer Analyseeinrichtung (2) die mit dem Erregungswellen-Detektor verbunden und ausgebildet ist, das erstes Signal $S_1(t)$ und zweites Signal $S_2(t)$ zu analysieren, wobei die Analyseeinrichtung ausgebildet ist, einen Unterschied zwischen einer Signalform des ersten Signals $S_1(t)$ und zweiten Signals $S_2(t)$ zu erfassen, **dadurch gekennzeichnet, dass** die Analyseeinrichtung eine Dämpfungs-Analyse-Einheit (14) umfasst, die entweder mit einer Fourier-Analyse-Einheit (11) verbunden ist und ausgebildet ist, eine Dämpfung δ(w) einer Fourier-Komponente der Fourier-Analyse zwischen den Punkten $r_1$ und $r_2$ zu ermitteln, wobei die Fourier-Analyse-Einheit ausgebildet ist, eine Fourier-Analyse durchzuführen, wobei für die Funktionen

$$f(wt) = \exp(iwt) \text{ einzusetzen ist und } \quad C_1(w) = \int_{-\infty}^{\infty} S_1(t)\exp(-iwt)dt$$

und

$$C_2(w) = \int_{-\infty}^{\infty} S_2(t)\exp(-iwt)dt$$

gilt
oder mit einer Wavefet-Analyse-Einheit (12) verbunder: ist und ausgebildet ist, eine Dämpfung

$$\delta(a) = \int_{-\infty}^{\infty} s_{a,2}^2(t)dt \Big/ \int_{-\infty}^{\infty} s_{a,1}^2(t)dt \quad \text{der Wavelet-Komponente } s_a(t) \text{ zwischen den Punkten } r_1 \text{ und } r_2 \text{ zu ermitteln,}$$

wobei die Wavelet-Analyse-Einheit ausgebildet ist, für die Signale $S_1(t)$ und $S_2(t)$ die Wavelet-Komponenten $s_{a,1}(t)$ und $s_{a,2}(t)$ zu berechnen, die durch

$$s_{a,1}(t) = \int_{-\infty}^{\infty} a^{-1/2} C_1(a,b) \Psi\left(\frac{t-b}{a}\right) db \quad \text{und} \quad s_{a,2}(t) = \int_{-\infty}^{\infty} a^{-1/2} C_2(a,b) \Psi\left(\frac{t-b}{a}\right) db$$

gegeben sind, $\Psi$ $((t-b)/a)$ sind hierbei Wavelets und $C_1(a,b)$ sowie $C_2(a,b)$ die jeweiligen Wavelet-Transformierten

$$C_1(a,b) = \int_{-\infty}^{\infty} a^{-1/2} S_1(t) \Psi((t-b)/a) dt \quad \text{und} \quad C_2(a,b) = \int_{-\infty}^{\infty} a^{-1/2} S_2(t) \Psi((t-b)/a) dt$$

von $S_1(t)$ und $S_2(t)$.

2. Vorrichtung nach Anspruch 1, **dadurch gekennzeichnet, dass** die Analyseeinrichtung ausgebildet ist, das erste $S_1(t)$ und zweite Signal $S_2(t)$ durch eine Überlagerung einer Menge von Funktionen f(wt) mit $w \in R$ darzustellen, wobei

$$S_1(t) = \int_{-\infty}^{\infty} C_1(w) f(wt) dw \quad \text{und} \quad S_2(t) = \int_{-\infty}^{\infty} C_2(w) f(wt) dw$$

entspricht.

3. Vorrichtung nach Anspruch 1, **dadurch gekennzeichnet, dass** die Analyseeinrichtung eine Geschwindigkeits-Analyse-Einheit (13) umfasst, die mit der Fourier-Analyse-Einheit verbunden ist und ausgebildet ist, eine Phasengeschwindigkeit $v_p(w)$ einer Fourier-Komponente der Fourier-Analyse zu ermitteln.

4. Vorrichtung nach Anspruch 1, **dadurch gekennzeichnet, dass** die Geschwindigkeits-Analyse-Einheit mit der Wavelet-Analyse-Einheit verbunden ist und ausgebildet ist, eine Phasengeschwindigkeit $v_p(a)$ der Wavelet-Komponente $s_a(t)$ mittels $v_p(a)=|r_2-r_1|/(t_{a,2}-t_{a,1})$, wobei $s_{a,1}(t_{a,1})=0$ und $s_{a,2}(t_{a,2}) = 0$ sind, zu ermitteln.

5. Vorrichtung nach Anspruch 4, **dadurch gekennzeichnet, dass** die Geschwindigkeits-Analyse-Einheit mit der Wavelet-Analyse-Einheit verbunden ist und ausgebildet ist, eine Gruppengeschwindigkeit $v_g(a)$ der Wavelet-Komponente $s_a(t)$ mittels $v_g(a)= |r_2-r_1|/(\tau_{a,2}-\tau_{a,1})$ mit $\max(A_{a,1}(t))=A_{a,1}(\tau_{a,1})$ von $A_{a,1}(t)$ und $\max(A_{a,2}(t))=A_{a,2}(\tau_{a,2})$ von $A_{a,2}(t)$ zu ermitteln, wobei $A_{a,1}(t)$ und $A_{a,2}(t)$ jeweils die Einhüllenden $A_{a,1} = \left[ s_{a,1}^2(t) + \hat{s}_{a,1}^2(t) \right]^{1/2}$ und $A_{a,2} = \left[ s_{a,2}^2(t) + \hat{s}_{a,2}^2(t) \right]^{1/2}$ der Wavelet-Komponenten darstellen und

$$\hat{s}_{a,1}(t) = -\pi^{-1} \int_{-\infty}^{\infty} \frac{s_{a,1}(t)}{\tau - t} d\tau \quad \text{und} \quad \hat{s}_{a,2}(t) = -\pi^{-1} \int_{-\infty}^{\infty} \frac{s_{a,2}(t)}{\tau - t} d\tau$$

entsprechen.

6. Vorrichtung nach Anspruch 1 und 5, **dadurch gekennzeichnet, dass** die Analyseeinrichtung eine Refraktionsindex-Analyse-Einheit umfasst, die mit der Geschwindigkeits-Analyse-Einheit verbunden ist und ausgebildet ist, einen Refraktionsindex n(a) mittels $n(a)=v_g(a)/v_p(a)$ zu ermitteln.

7. Vorrichtung nach Anspruch 1, **dadurch gekennzeichnet, dass** der Erregungswellendetektor eine erste und zweite Elektrode zum Erfassen des ersten Signals $S_1(t)$ und zweiten Signals $S_2(t)$ aufweist.

8. Vorrichtung nach Anspruch 7, **dadurch gekennzeichnet, dass** die erste und zweite Elektrode ausgebildet sind, endokardial oder epikardial plaziert zu werden.

9. Vorrichtung nach Anspruch 1, **gekennzeichnet durch** einen Signalspeicher, der mit dem Erregungswellendetektor und der Analyseeinrichtung verbunden und ausgebildet ist, das erste und zweite Signal zwischenzuspeichern.

10. Verfahren zum Betreiben einer Analysevorrichtung (2) nach einem der vorstehenden Ansprüche, mit dem Schritt Erfassen einer elektrischen Erregungswelle an einem ersten $r_1$ und zweiten Punkt $r_2$ des Myokards als erstes Signal $S_1(t)$ und zweites Signal $S_2(t)$, sowie einem Schritt des Berechnens eines Unterschieds zwischen einer Signalform des ersten Signals $S_1(t)$ und zweiten Signals $S_2(t)$, gekennzeichnet entweder

durch den Schritt Darstellen des ersten Signals $S_1(t)$ und zweiten Signals $S_2(t)$ durch eine Überlagerung einer Menge von Funktionen {f(wt)} mit w∈ R, wobei

$$S_1(t) = \int_{-\infty}^{\infty} C_1(w) f(wt)\, dw \ \text{ und } \ S_2(t) = \int_{-\infty}^{\infty} C_2(w) f(wt)\, dw$$

entspricht
und den Schritt Ermitteln einer Dämpfung δ(w) einer Fourier-Komponente der Fourier-Analyse zwischen den Punkten r1 und r2

oder

durch den Schritt Einsetzen von Wavelet-Komponenten $s_a(t)$ für die Signale $S_1(t)$ und $S_2(t)$, wobei

$$S_1(t) = \sum_{k=-\infty}^{\infty} s_{a_k,1}(t) \ \text{ und } \ S_2(t) = \sum_{k=-\infty}^{\infty} s_{a_k,2}(t) \ \text{ entsprechen, die Wavelet-Komponenten durch}$$

$$s_{a_k,1}(t) = \int_{-\infty}^{\infty} a_k^{-1/2} C_1(a_k,b)\Psi\!\left(\frac{t-b}{a_k}\right) db \ \text{ und } \ s_{a_k,2}(t) = \int_{-\infty}^{\infty} a_k^{-1/2} C_2(a_k,b)\Psi\!\left(\frac{t-b}{a_k}\right) db$$

gegeben sind, Ψ((t-b)/a) Wavelets sind und $C_1(a,b)$ sowie $C_2(a,b)$ die jeweils entsprechende Wavelet-Transformierten

$$C_1(a,b) = \int_{-\infty}^{\infty} a^{-1/2} S_1(t)\Psi((t-b)/a)\, dt \ \text{ und } \ C_2(a,b) = \int_{-\infty}^{\infty} a^{-1/2} S_2(t)\Psi((t-b)/a)\, dt$$

von $S_1(t)$ und $S_2(t)$ darstellen

und den Schritt Ermitteln einer Dämpfung δ(a) der Wavelet-Komponenten $s_a(t)$ zwischen den Punkten $r_1$ und $r_2$

mittels $\delta(a) = \int_{-\infty}^{\infty} s_{a,2}^2(t)\, dt \left/ \int_{-\infty}^{\infty} s_{a,1}^2(t)\, dt \right.$ .

11. Verfahren nach Anspruch 10, **gekennzeichnet durch** den Schritt Ermitteln einer Phasengeschwindigkeit $v_p(w)$ einer Fourier-Komponente der Fourier-Analyse.

12. Verfahren nach Anspruch 10, **gekennzeichnet durch** den Schritt Ermitteln einer Phasengeschwindigkeit $v_p(a)$ der Wavelet-Komponenten $s_a(t)$ zwischen den Punkten $r_1$ und $r_2$ mittels $v_p(a)=|r_2-r_1|/(t_{a,2}-t_{a,1})$ mit $s_{a,1}(t_{a,1}) = 0$ und $s_{a,2}(t_{a,2}) = 0$.

13. Verfahren nach einem der Ansprüche 10 oder 12, **gekennzeichnet durch** den Schritt Ermitteln einer Gruppengeschwindigkeit $v_g(a)$ der Wavelet-Komponenten $s_a(t)$ mittels $v_g(a)= |r_2-r_1|/(\tau_{a,2}-\tau_{a,1})$ mit $\max(A_{a,1}(t))=A_{a,1}(\tau_{a,1})$ von

$A_{a,1}(t)$ und $\max(A_{a,2}(t)) = A_{a,2}(\tau_{a,2})$ von $A_{a,2}(t)$ zu ermitteln, wobei $A_{a,1}(t)$ und $A_{a,2}(t)$ jeweils Einhüllende

$$A_{a,1} = \left[ s_{a,1}^2(t) + \hat{s}_{a,1}^2(t) \right]^{1/2} \text{ und } A_{a,2} = \left[ s_{a,2}^2(t) + \hat{s}_{a,2}^2(t) \right]^{1/2} \text{ mit } \hat{s}_{a,1}(t) = -\pi^{-1} \int\limits_{-\infty}^{\infty} \frac{s_{a,1}(t)}{\tau - t} d\tau$$

und

$$\hat{s}_{a,2}(t) = -\pi^{-1} \int\limits_{-\infty}^{\infty} \frac{s_{a,2}(t)}{\tau - t} d\tau$$

darstellen.

14. Verfahren nach Anspruch 12 und 13, **gekennzeichnet durch** den Schritt Berechnen eines Refraktionsindex n(a) mittels $n(a) = v_g(a)/v_p(a)$.

**Claims**

1. A device for characterising a condition of a myocardium with an excitation wave detector (1) that is designed for the purpose of recording an electrical excitation wave propagating through the myocardium at a first point $r_1$ and a second point $r_2$ of the myocardium as a first signal $S_1(t)$ and a second signal $S_2(t)$, and an analysis device (2) that is connected and designed with the excitation wave detector to analyse the first $S_1(t)$ and second $S_2(t)$ signal, wherein the analysis device is designed to record a difference between a signal form of the first signal $S_1(t)$ and the second signal $S_2(t)$,
   **characterised in that** the analysis device comprises a damping analysis unit (14) that
   either
   is connected with a Fourier analysis unit (11) and designed to determine a damping $\delta(w)$ of a Fourier component of the Fourier analysis between the points $r_1$ and $r_2$, wherein
   the Fourier analysis unit is designed to perform a Fourier analysis, wherein
   for the functions f(wt) = exp(iwt) is to be inserted, and

$$C_1(w) = \int\limits_{-\infty}^{\infty} S_1(t) \exp - iwt) dt \text{ and } C_2(w) = \int\limits_{-\infty}^{\infty} S_2(t) \exp - iwt) dt$$

   or
   is connected with a wavelet analysis unit (12) and designed to determine a damping

$$\delta(a) = \int\limits_{-\infty}^{\infty} s_{a,2}^2(t) dt / \int\limits_{-\infty}^{\infty} s_{a,1}^2(t) dt$$

   of the wavelet component $s_a(t)$ between the points $r_1$ and $r_2$, wherein the wavelet analysis unit is designed to calculate for the signals $S_1(t)$ and $S_2(t)$ the wavelet components $s_{a,1}(t)$ and $s_{a,2}(t)$, which are given by

$$( s_{a,1}(t) = \int\limits_{-\infty}^{\infty} a^{-1/2} C_1(a,b) \Psi\left( \frac{t-b}{a} \right) db \text{ and } s_{a,2}(t) = \int\limits_{-\infty}^{\infty} a^{-1/2} C_2(a,b) \Psi\left( \frac{t-b}{a} \right) db ,$$

   where $\Psi((t-b)/a)$

are wavelets and $C_1(a,b)$ and $C_2(a,b)$ are the respective transformed wavelets

$$C_1(a,b) = \int_{-\infty}^{\infty} a^{-1/2} S_1(t) \Psi((t-b)/a) dt \quad \text{and} \quad C_2(a,b) = \int_{-\infty}^{\infty} a^{-1/2} S_2(t) \Psi((t-b)/a) dt$$

of $S_1(t)$ and $S_2(t)$.

2. The device according to Claim 1,
**characterised in that** the analysis device is designed to represent the first $S_1(t)$ and second signal $S_2(t)$ by overlaying a number of functions f(wt) with w∈R, wherein

$$S_1(t) = \int_{-\infty}^{\infty} C_1(w) f(wt) dw \quad \text{and} \quad S_2(t) = \int_{-\infty}^{\infty} C_2(w) f(wt) dw \ .$$

3. The device according to Claim 1,
**characterised in that** the analysis device comprises a velocity analysis unit (13) that is connected and designed with the Fourier analysis unit to determine a phase velocity $v_p(w)$ of a Fourier component of the Fourier analysis.

4. The device according to Claim 1,
**characterised in that** the velocity analysis unit is connected with the wavelet analysis unit and designed to determine a phase velocity $v_p(a)$ of the wavelet component $s_a(t)$ by means of $v_p(a)=|r_2-r_1-|/(t_{a,2}-t_{a,1})$, wherein $s_{a,1}(t_{a,1})=0$ and $s_{a,2}(t_{a,2})=0$.

5. The device according to Claim 4,
**characterised in that** the velocity analysis unit is connected with the wavelet analysis unit and designed to determine a group velocity $v_g(a)$ of the wavelet component $s_a(t)$ by means of $v_g(a)=|r_2-r_1-|/(\tau_{a,2}-\tau_{a,1})$ with $\max(A_{a,1}(t))=A_{a,1}(\tau_{a,1})$ of $A_{a,1}(t)$ and $\max(A_{a,2}(t))=A_{a,2}(\tau_{a,2})$ of $A_{a,2}(t)$, wherein $A_{a,1}(t)$ and $A_{a,2}(t)$ respectively represent the envelopes

$$A_{a,1} = \left[ s_{a,1}^2(t) + \hat{s}_{a,1}^{\,2}(t) \right]^{1/2} \quad \text{and} \quad A_{a,2} = \left[ s_{a,2}^2(t) + \hat{s}_{a,2}^{\,2}(t) \right]^{1/2} \quad \text{of the wavelet components and}$$

$$\hat{s}_{a,1}(t) = -\pi^{-1} \int_{-\infty}^{\infty} \frac{s_{a,1}(t)}{\tau - t} d\tau \quad \text{and} \quad \hat{s}_{a,2}(t) = -\pi^{-1} \int_{-\infty}^{\infty} \frac{s_{a,2}(t)}{\tau - t} d\tau \ .$$

6. The device according to Claims 1 and 5,
**characterised in that** the analysis device comprises a refraction analysis unit (13) that is connected with the velocity analysis unit and designed to determine a refraction index n(a) by means *of n(a) = $v_s(a)/v_P(a)$.*

7. The device according to Claim 1,
**characterised in that** the excitation wave detector has a first and second electrode to record the first signal $S_1(t)$ and second signal $S_2(t)$.

8. The device according to Claim 7,
**characterised in that** the first and second electrode are designed to be placed in an endocardial or epicardial position.

9. The device according to Claim 1,

**characterised by** a signal store that is connected and designed with the excitation wave detector and the analysis device to provide intermediate storage for the first and second signal.

10. A method for the operation of an analysis device (2) according to one of the previous claims, with the step of recording an electrical excitation wave at a first $r_1$ and second point $r_2$ of the myocardium as a first signal $S_1(t)$ and a second signal $S_2(t)$, as well as a step of calculating a difference between a signal form of the first signal $S_1(t)$ and second signal $S_2(t)$, **characterised**
either
by the step of representing the first $S_1(t)$ and second signal $S_2(t)$ by overlaying a number of functions f(wt) with $w \in R$, wherein

$$S_1(t) = \int_{-\infty}^{\infty} C_1(w) f(wt) dw \quad \text{and} \quad S_2(t) = \int_{-\infty}^{\infty} C_2(w) f(wt) dw$$

and the step of determining a damping $\delta(w)$ of a Fourier component of the Fourier analysis between the points $r_1$ and $r_2$
or
by the step of inserting wavelet components $s_a(t)$ for the signals $S_1(t)$ and $S_2(t)$, wherein

$$S_1(t) = \sum_{k=-\infty}^{\infty} s_{a_k,1}(t) \quad \text{and} \quad S_2(t) = \sum_{k=-\infty}^{\infty} s_{a_k,2}(t)(a_k = 2^k),$$

the wavelet components are given by

$$s_{a_k,1}(t) = \int_{-\infty}^{\infty} a_k^{-1/2} C_1(a_k,b) \Psi\left(\frac{t-b}{a_k}\right) db \quad \text{and} \quad s_{a_k,2}(t) = \int_{-\infty}^{\infty} a_k^{-1/2} C_2(a_k,b) \Psi\left(\frac{t-b}{a_k}\right) db,$$

where $\Psi(t-b)/a)$ are wavelets and $C_1(a,b)$ and $C_2(a,b)$ represent the respective transformed wavelets

$$C_1(a,b) = \int_{-\infty}^{\infty} a^{-1/2} S_1(t) \Psi(t-b)/a) dt \quad \text{and} \quad C_2(a,b) = \int_{-\infty}^{\infty} a^{-1/2} S_2(t) \Psi(t-b)/a) dt$$

of $S_1(t)$ and $S_2(t)$, and the step of determining a damping $\delta(w)$ of a wavelet component $s_a(t)$ between the points $r_1$ and $r_2$ by means of

$$\delta(a) = \int_{-\infty}^{\infty} s_{a,2}^2(t) dt / \int_{-\infty}^{\infty} s_{a,1}^2(t) dt.$$

11. The method according to Claim 10,
**characterised by** the step of determining a phase velocity $v_p(w)$ of a Fourier component of the Fourier analysis.

12. The method according to Claim 10,
**characterised by** the step of determining a phase velocity $v_p(a)$ of a wavelet component $s_a(t)$ between the points $r_1$ and $r_2$ by means of

$$V_p(a) = |r_2 - r_1|/(t_{a,2} - t_{a,1}) \text{ with } s_{a,1}(t_{a,1}) = 0 \text{ and } s_{a,2}(t_{a,2}) = 0.$$

13. The method according to one of the Claims 10 or 12,
**characterised by** the step of determining a group velocity $v_g(a)$ of the wavelet component $s_a(t)$ by means of $v_g(a)$ $=|r_2-r_1|/(\tau_{a,2}-\tau_{a,1})$ with $\max(A_{a,1}(t)) = A_{a,1}(\tau_{a,1})$ of $A_{a,1}(t)$ and $\max(A_{a,2}(t)) = A_{a,2}(\tau_{a,2})$ of $A_{a,2}(t)$ of $A_{a,2}(t)$ wherein $A_{a,1}(t)$ und $A_{a,2}(t)$ respectively represent envelopes

$$A_{a,1} = \left[ s_{a,1}^2(t) + \hat{s}_{a,1}^2(t) \right]^{1/2} \qquad \text{and} \qquad A_{a,2} = \left[ s_{a,2}^2(t) + \hat{s}_{a,2}^2(t) \right]^{1/2}$$

with

$$\hat{s}_{a,1}(t) = -\pi^{-1} \int_{-\infty}^{\infty} \frac{s_{a,1}(t)}{\tau - t} d\tau \quad \text{and} \quad \hat{s}_{a,2}(t) = -\pi^{-1} \int_{-\infty}^{\infty} \frac{s_{a,2}(t)}{\tau - t} d\tau$$

14. The method according to Claim 12 and 13, **characterised by** the step of calculating a refractive index n(a) by means of $n(a)=v_g(a)/v_p(a)$.

**Revendications**

1. Dispositif pour caractériser un état d'un myocarde avec un détecteur d'ondes d'excitation (1), qui est conçu pour détecter une onde d'excitation électrique, se propageant à travers le myocarde, sur un premier point $r_1$ et un second point $r_2$ du myocarde comme premier signal $S_1(t)$ et comme second signal $S_2(t)$, et un dispositif d'analyse (2), qui est relié au détecteur d'ondes d'excitation et est conçu pour analyser le premier signal $S_1(t)$ et le second signal $S_2(t)$, le dispositif d'analyse étant conçu pour détecter une différence entre une forme de signal du premier signal $S_1(t)$ et du second signal $S_2(t)$, **caractérisé en ce que** le dispositif d'analyse comprend une unité d'analyse d'amortissement, qui est reliée à une unité d'analyse de Fourier et est conçue pour déterminer un amortissement δ(w) d'une composante de Fourier de l'analyse de Fourier entre les points $r_1$ et $r_2$, l'unité de l'analyse de Fourier étant conçue pour effectuer une analyse de Fourier, sachant qu'on doit utiliser pour les fonctions

$$f(wt) = \exp(iwt) \text{ et qu'on a } C_1(w) = \int_{-\infty}^{\infty} S_1(t) \exp - iwt) dt$$

et

$$C_2(w) = \int_{-\infty}^{\infty} S_2(t) \exp - iwt) dt$$

ou étant reliée à une unité d'analyse d'ondelette (12), et étant conçue pour déterminer un amortissement

$$\delta(a) = \int_{-\infty}^{\infty} s_{a,2}^2(t)dt \left/ \int_{-\infty}^{\infty} s_{a,1}^2(t)dt \right.$$ de la composante d'ondelette $S_a$(t) entre les points $r_1$ et $r_2$, l'unité d'analyse d'ondelette étant conçue pour calculer pour les signaux $S_1$(t) et $S_2$(t) les composantes d'ondelette $S_{a,1}$(t) et $S_{a,2}$(t), qui sont données par

$$( \, s_{a,1}(t) = \int_{-\infty}^{\infty} a^{-1/2} C_1(a,b) \Psi\left(\frac{t-b}{a}\right) db \qquad \text{et} \qquad s_{a,2}(t) = \int_{-\infty}^{\infty} a^{-1/2} C_2(a,b) \Psi\left(\frac{t-b}{a}\right) db \, ,$$

$\Psi((t\text{-}b)/a)$ sont des ondelettes et $C_1$(a, b) et $C_2$(a, b) les transformées d'ondelette respectives

$$C_1(a,b) = \int_{-\infty}^{\infty} a^{-1/2} S_1(t) \Psi((t-b)/a) dt \quad \text{et} \quad C_2(a,b) = \int_{-\infty}^{\infty} a^{-1/2} S_2(t) \Psi((t-b)/a) dt \quad \text{de } S_1(t) \text{ et}$$

$S_2$(t).

2. Dispositif selon la revendication 1, **caractérisé en ce que** le dispositif d'analyse est conçu pour représenter le premier signal $S_1$(t) et le second signal $S_2$(t) par une superposition d'une quantité de fonctions f(wt) avec $w \in R$, sachant que

$$S_1(t) = \int_{-\infty}^{\infty} C_1(w) f(wt) dw \text{ et } S_2(t) = \int_{-\infty}^{\infty} C_2(w) f(wt) dw$$

3. Dispositif selon la revendication 1, **caractérisé en ce que** le dispositif d'analyse comprend une unité d'analyse de vitesse (13), qui est reliée à l'unité d'analyse de Fourier et est conçue pour déterminer une vitesse de phase $v_p$(wt) d'une composante de Fourier de l'analyse de Fourier.

4. Dispositif selon la revendication 1, **caractérisé en ce que** l'unité d'analyse de vitesse est reliée à l'unité d'analyse d'ondelette et est conçue pour déterminer une vitesse de phase $v_p$(a) de la composante d'ondelette $s_a$(t) au moyen de $v_p(a)=|r_2\text{-}r_1\text{-}|/(t_{a,2}\text{-}t_{a,1})$, sachant que $s_{a,1}(t_{a,1})=0$ et $s_{a,2}(t_{a,2})=0$.

5. Dispositif selon la revendication 4, **caractérisé en ce que** l'unité d'analyse de vitesse est reliée à l'unité d'analyse d'ondelette et est conçue pour déterminer une vitesse de groupe $v_g$(a) de la composante d'ondelette $S_a$(t) au moyen de $v_g(a)=|r_2\text{-}r_1\text{-}|/(\tau_{a,2}\text{-}\tau_{a,1})$ avec max($A_{a,1}$(t))=$A_{a,1}(\tau_{a,1})$ de $A_{a,1}(\tau)$ et max($A_{a,2}$(t))=$A_{a,2}(\tau_{a,2})$ de $A_{a,2}(\tau)$, sachant que $A_{a,1}(\tau)$ et $A_{a,2}(\tau)$ représentent respectivement les enveloppantes

$$A_{a,1} = \left[ s_{a,1}^2(t) + \overset{\wedge}{s}{}_{a,1}^{\,2}(t) \right]^{1/2} \qquad \text{et} \qquad A_{a,2} = \left[ s_{a,2}^2(t) + \overset{\wedge}{s}{}_{a,2}^{\,2}(t) \right]^{1/2} \quad \text{des composantes d'ondelette et sachant}$$

qu'on a $\quad \overset{\wedge}{s}_{a,1}(t) = -\pi^{-1} \int_{-\infty}^{\infty} \frac{s_{a,1}(t)}{\tau - t} d\tau \quad$ et $\quad \overset{\wedge}{s}_{a,2}(t) = -\pi^{-1} \int_{-\infty}^{\infty} \frac{s_{a,2}(t)}{\tau - t} d\tau \, .$

6. Dispositif selon les revendications 1 et 5, **caractérisé en ce que** le dispositif d'analyse comprend une unité d'analyse d'indice de réfraction, qui est reliée à l'unité d'analyse de vitesse et est conçue pour déterminer un indice de réfraction n(a) au moyen de $n(a) = v_s(a)/v_P(a)$.

**7.** Dispositif selon la revendication 1, **caractérisé en ce que** le détecteur d'ondes d'excitation présente une première et une seconde électrodes pour la détection du premier signal $S_1(t)$ et du second signal $S_2(t)$.

**8.** Dispositif selon la revendication 7, **caractérisé en ce que** la première et la seconde électrodes sont conçues pour être placées de façon endocardiale ou épicardiale.

**9.** Dispositif selon la revendication 1, **caractérisé par** une mémoire de signal, qui est relié à un détecteur d'ondes d'excitation et au dispositif d'analyse et est conçu pour stocker provisoirement le premier et le second signaux.

**10.** Procédé pour l'exploitation d'un dispositif d'analyse (2) selon l'une quelconque des revendications précédentes, avec l'étape détection d'une onde d'excitation électrique sur un premier point $r_1$ et second point $r_2$ du myocarde comme premier signal $S_1(t)$ et second signal $S_2(t)$ et une étape du calcul d'une différence entre une forme de signal du premier signal $S_1(t)$ et du second signal $S_2(t)$, **caractérisé** soit par l'étape représentation du premier signal $S_1(t)$ et du second signal $S_2(t)$ par une superposition d'une quantité de fonctions {f(wt)} avec $w \in R$, sachant qu'on a

$$S_1(t) = \int_{-\infty}^{\infty} C_1(w) f(wt) dw \text{ et } S_2(t) = \int_{-\infty}^{\infty} C_2(w) f(wt) dw$$

et par l'étape calcul d'un amortissement $\delta(w)$ d'une composante de Fourier de l'analyse de Fourier entre les points $r_1$ et $r_2$
ou
par l'étape utilisation de composantes d'ondelette $s_a(t)$ pour les signaux $S_1(t)$ et $S_2(t)$, sachant qu'on a

$$S_1(t) = \sum_{k=-\infty}^{\infty} s_{a_k,1}(t) \quad \text{et} \quad S_2(t) = \sum_{k=-\infty}^{\infty} s_{a_k,2}(t)(a_k = 2^k) \,, \text{ les composantes d'ondelette étant données par}$$

$$s_{a_k,1}(t) = \int_{-\infty}^{\infty} a_k^{-1/2} C_1(a_k,b) \Psi\left(\frac{t-b}{a_k}\right) db \text{ et } s_{a_k,2}(t) = \int_{-\infty}^{\infty} a_k^{-1/2} C_2(a_k,b) \Psi\left(\frac{t-b}{a_k}\right) db$$

$\Psi(t-b)/a)$ étant des ondelettes et $C_1(a,b)$ et $C_2(a,b)$ représentant les transformées d'ondelette respectivement correspondantes

$$C_1(a,b) = \int_{-\infty}^{\infty} a^{-1/2} S_1(t) \Psi(t-b)/a) dt \text{ et } C_2(a,b) = \int_{-\infty}^{\infty} a^{-1/2} S_2(t) \Psi(t-b)/a) dt$$

de $S_1(t)$ et de $S_2(t)$
et l'étape calcul d'un amortissement $\delta(a)$ des composantes d'ondelette $S_a(t)$ entre les points $r_1$ et $r_2$ au moyen de

$$\delta(a) = \int_{-\infty}^{\infty} s_{a,2}^2(t) dt / \int_{-\infty}^{\infty} s_{a,1}^2(t) dt \,.$$

**11.** Procédé selon la revendication 10, **caractérisé par** l'étape calcul d'une vitesse de phase $v_p(w)$ d'une composante de Fourier de l'analyse de Fourier.

**12.** Procédé selon la revendication 10, **caractérisé par** l'étape calcul d'une vitesse de phase $v_p(a)$ des composantes d'ondelette $S_a(t)$ entre les points $r_1$ et $r_2$ au moyen de

$$V_p(a) = |r_2\text{-}r_1|/(t_{a,2}\text{-}t_{a,1}) \text{ avec } s_{a,1}(t_{a,1}) = 0 \text{ et } s_{a,2}(t_{a,2}) = 0$$

**13.** Procédé selon l'une quelconque des revendications 10 ou 12, **caractérisé par** l'étape calcul d'une vitesse de groupe $v_g(a)$ des composantes d'ondelette $s_a(t)$ au moyen de $v_g(a) = |r_2\text{-}r_1|/(\tau_{a,2}\text{-}\tau_{a,1})$ avec $\max(A_{a,1}(t)) = A_{a,1}(\tau_{a,1})$ de $A_{a,1}(t)$ et $\max(A_{a,2}(t)) = A_{a,2}(\tau_{a,2})$ de $A_{a,2}(t)$ de $A_{a,2}(t)$, sachant que $A_{a,1}(t)$ et $A_{a,2}(t)$ représentent respectivement des enveloppantes

$$A_{a,1} = \left[ s_{a,1}^2(t) + \overset{\wedge}{s}_{a,1}^2(t) \right]^{1/2} \qquad \text{et} \qquad A_{a,2} = \left[ s_{a,2}^2(t) + \overset{\wedge}{s}_{a,2}^2(t) \right]^{1/2}$$

avec

$$\overset{\wedge}{s}_{a,1}(t) = -\pi^{-1} \int_{-\infty}^{\infty} \frac{s_{a,1}(t)}{\tau - t} d\tau \text{ et } \overset{\wedge}{s}_{a,2}(t) = -\pi^{-1} \int_{-\infty}^{\infty} \frac{s_{a,2}(t)}{\tau - t} d\tau$$

**14.** Procédé selon les revendications 12 et 13, **caractérisé par** l'étape calcul d'un indice de réfraction n(a) au moyen de $n(a) = v_g(a)/v_p(a)$.

Fig. 1

Fig. 2

$s_\alpha(t)$

$A_\alpha(t)$

t

t

**Fig. 3**

$s_{a,1}(t)$ — channel 1

$s_{a,2}(t)$ — channel 2

t

t

$t_{a,2} - t_{a,1}$

**Fig. 4**

$s_{a,1}(t)$ — channel 1

$s_{a,2}(t)$ — channel 2

t

t

$\tau_{a,2} - \tau_{a,1}$

**Fig. 5**

**IN DER BESCHREIBUNG AUFGEFÜHRTE DOKUMENTE**

**In der Beschreibung aufgeführte Patentdokumente**

- EP 0947215 A